# EUROPÄISCHE PATENTSCHRIFT

(11) **EP 0 496 954 B1**
(45) Veröffentlichungstag und Bekanntmachung des Hinweises auf die Patenterteilung: **11.05.1994**
(21) Anmeldenummer: 91118281.4
(22) Anmeldetag: 26.10.1991
(51) Int. Cl.: A61B 17/58

(54) **Knochennagel zur Versorgung von Oberarmfrakturen**
Bone pin for treatment of de upper arm
Clou centro-médullaire destiné au traitement du bras

(30) Priorität: 30.01.1991 DE 9101035 U
(43) Veröffentlichungstag der Anmeldung: 05.08.1992
(73) Patentinhaber: Howmedica GmbH, D-24232 Schönkirchen (DE)
(72) Erfinder: Seidel, Hartmut Richard Alfred, Dr. med., W-2000 Hamburg 52 (DE); Speitling, Andreas Werner, Dr.-Ing., W-2300 Kiel 1 (DE)
(74) Vertreter: Patentanwälte Hauck, Graalfs, Wehnert, Döring, Siemons

(56) Entgegenhaltungen:
- EP-A- 0 226 701
- DE-A- 2 701 279
- DE-A- 3 146 065
- FR-A- 2 141 020
- US-A- 3 760 802

## Beschreibung

Gegenstand der Erfindung ist ein Knochennagel zur Versorgung von Oberarmfrakturen nach dem Oberbegriff des Anspruchs 1.

Eine Schraube der eingangs genannten Art, deren Gewindeabschnitt auch in ein Innengewinde in Erhebungen am Innenumfang des Schaftes eingreifen kann, ist schon aus der EP-A-0 226 701 bekannt. Dabei ist der Spreizkörper vom Kopf der Schraube gebildet, der sich zum Schraubenschaft hin konisch verjüngen kann. Beim Eindrehen der Schraube durch Ansetzen eines Werkzeuges am freien Ende des Schraubenschaftes spreizt der Kopf das geschlitzte Ende des Schaftes radial auseinander, wodurch eine Verdübelung mit dem Knochen erfolgt. Beim Auseinanderspreizen rotiert der Kopf über den Innenumfang des Schaftes, ggfs. über Erhebungen, die einen von distal nach proximal abnehmenden Innenradius definieren. Das Eindrehmoment der Schraube ist deshalb zu einem erheblichen Teil durch das auf den Kopf einwirkende Reibmoment bestimmt, welches bei Anlage an Erhebungen punktuell angreift. Das hohe Drehmoment erschwert das Spreizen des distalen Nagelendes und kann Lageveränderungen verursachen. Außerdem beeinträchtigt es die Kontrolle des Spreizfortschrittes und der damit auf den Knochen ausgeübten Spreizkraft durch den Chirurgen. Ebenso erschwert es das Lösen der Schraube und dessen Kontrollierbarkeit. Gleitet der Kopf auf Erhebungen des Nagels, kann die Kontrolle des Spreizfortschrittes außerdem durch unregelmäßige Momentenschwankungen gestört sein.

Davon ausgehend liegt der Erfindung die Aufgabe zugrunde, einen verbesserten Knochennagel zu schaffen, der bei verringertem und konstantem Drehmoment der Spreizschraube eine verbesserte Kontrolle über den Spreizfortschritt ermöglicht.

Zur Lösung dieser Aufgabe ist erfindungsgemäß bei einem eingangs genannten Knochennagel vorgesehen, daß der Spreizkörper am Kopf der Schraube abgestützt und relativ zu diesem drehbar ist.

Auch beim erfindungsgemäßen Knochennagel wird der vorzugsweise sich zum Schraubenschaft hin verjüngende Spreizkörper durch Drehen der Schraube axial entlang des Schaftes verschoben, so daß er beim Einziehen ein Aufspreizen des geschlitzten Endes bewirkt und beim Ausdrücken ein radiales Zusammenziehen desselben ermöglicht. Im Unterschied zum vorbekannten Knochennagel ist jedoch der Spreizkörper relativ zum Kopf der Schraube drehbar. Wenn er von der Schraube gegen den Schaft gezogen wird, dreht er deshalb nicht mehr mit, sondern bewegt sich nur noch in Schaftrichtung. Infolgedessen entfällt ein zusätzliches und gegebenenfalls unregelmäßiges Drehmoment infolge eines Rotierens des Spreizkörpers bei Anlage am Schaft. Statt dessen entsteht zwar ein Reibmoment aufgrund seiner Abstützung am Kopf; dieses kann jedoch durch geeignete Ausbildung und Bemessung der Stützflächen relativ gering gehalten werden, zumal die Reibkraft relativ nahe an der Schaftachse angreift. Als Kopf der Schraube ist auch ein auf dem Schraubenschaft ausgebildeter Absatz zum Abstützen des Spreizkörpers anzusehen. Bei gleichem Anzugsmoment der Schraube des erfindungsgemäßen Knochennagels wird gegenüber einem herkömmlichen Nagel ein deutlich erhöhtes Losdrehmoment des Nagels im Knochen erreicht. Aufgrund des deutlich verringerten Anteils des Reibmoments am Anzugsmoment kann der behandelnde Arzt den Spreizfortschritt besser kontrollieren und den Schraubvorgang abbrechen ohne den Knochen zu beschädigen, wenn der Nagel gerade ausreichend verdübelt ist.

Gemäß einer bevorzugten Ausgestaltung der Erfindung liegt der Spreizkörper mit einer radial nach innen vorspringenden Stützfläche an der dem Schraubenschaft zugewandten Rückseite des Kopfes an, wobei er unverlierbar zwischen Kopf und Schaft des Nagels gehalten ist.

Damit der Spreizkörper beim Herausdrehen der Schraube auch sicher aus dem geschlitzten Endbereich des Nagels herausgedrückt wird, übergreift er nach einer bevorzugten Weiterbildung die vom Schraubenschaft abgewandte Vorderseite des Kopfes mit einem radial nach innen vorspringenden Rand, der vorzugsweise als Einbördelung ausgebildet ist. Beim Herausdrehen der Schraube nimmt dann der Kopf den Spreizkörper zwangsläufig mit, so daß es sicher zu einer Aufhebung der Verdübelung kommt. Die Ausbildung des Randes als Einbördelung ist fertigungstechnisch besonders günstig.

Vorzugsweise ist der Kopf der Schraube mit axialem Spiel zwischen vorspringendem Rand und Stützfläche des Spreizkörpers eingesetzt, wodurch insbesondere eine klemmfreie Drehbarkeit sichergestellt ist. Außerdem ermöglicht das axiale Spiel ein Lösen der Schraube in zwei "Stufen", wobei zunächst die Vorspannung der Schraube und, nach deren Verschieben über den Spielweg, die Vorspannung des Spreizkörpers abgebaut wird.

Eine praktisch bevorzugte Weiterbildung sieht vor, daß der Spreizkörper am Umfang der Schraube, vorzugsweise am Umfang des Kopfes, spielarm geführt ist. Dies begünstigt ein Einziehen des Spreizkörpers in den geöffneten Endbereich des Nagels, ohne daß sich sein Unterrand an dem Stirnrand des Nagels abstützt.

Bevorzugt ist der Kopf der Schraube zylindrisch, wodurch sich insbesondere eine große Anlagefläche der Rückseite und umfangsseitige Führungsfläche ergeben.

Für ein sicheres Einziehen des Spreizkörpers in die distale Nagelöffnung sowie eine große Aufspreizbarkeit des geschlitzten Nagelendes kann er außen einen Konus haben, der sich zum Schraubenschaft hin verjüngt. Insbesondere aus den gleichen Gründen kann der hohle Schaft innen in Umfangsrichtung abwechselnd Erhebungen und Vertiefungen haben, wobei der durch die Erhebungen definierte Innenradius im geschlitzten Schaftbereich vom distalen zum proximalen Ende des Schaftes hin abnimmt.

Schließlich sieht eine Ausgestaltung der Erfindung vor, daß der Spreizkörper aus rostfreiem Stahl, insbesondere einem implantierbaren Chromnickelstahl oder ggfs. anderen Implantatwerkstoffen besteht.

Weitere Einzelheiten und Vorteile der Erfindung ergeben sich aus der nachfolgenden Beschreibung der zugehörigen Zeichnung, die einen bevorzugten Nagel gemäß der Erfindung zeigt. In der Zeichnung zeigen:
- Fig. 1: der Knochennagel mit aufgespreiztem distalen Ende in Seitenansicht;
- Fig. 2: distales Ende desselben Knochennagels in ungespreiztem Zustand in Seitenansicht;
- Fig. 3: derselbe Knochennagel entlang der Linie III-III der Fig. 2 geschnitten;
- Fig. 4: derselbe Knochennagel entlang der Linie IV-IV der Fig. 2 geschnitten;
- Fig. 5: das distale Ende desselben Knochennagels entlang der Linie V-V der Fig. 3 und 4 längsgeschnitten;
- Fig. 6: Schraube mit Spreizkörper zum Spreizen des distalen Nagelendes im Längsschnitt.
- Fig. 7: eine abgewandelte Schraube mit Spreizkörper.

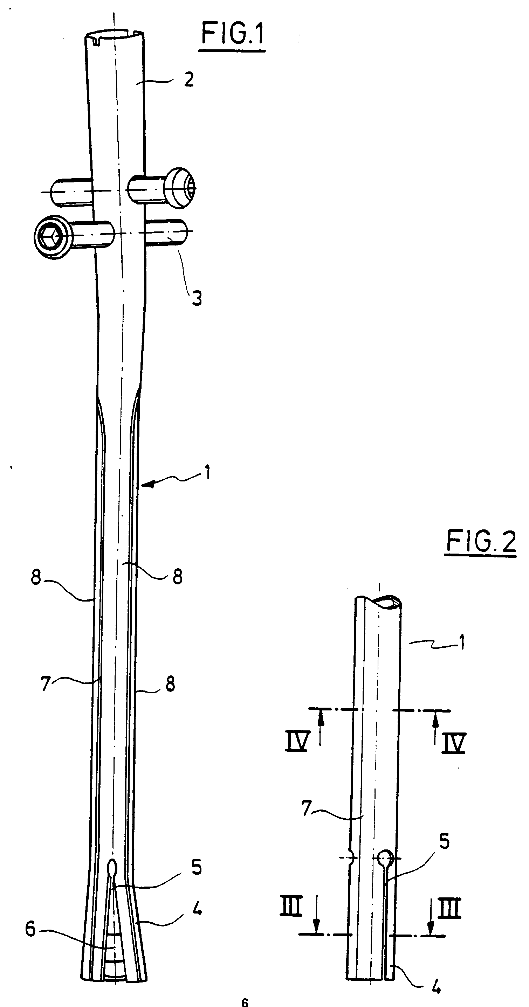

Die Fig. 1 zeigt einen Knochennagel 1, der nahe einem proximalen Ende 2 Querschrauben 3 zur Verankerung im Knochen aufnimmt. Nahe einem distalen Ende 4 ist er mit Längsschlitzen 5 versehen und mittels eines eingeschraubten Spreizkörpers 6 aufgespreizt. Über den Umfang des Schaftes 7 des Nagels 1 verteilt sind drei Erhebungen 8 angeordnet.

Wie der Fig. 2 entnehmbar ist, ist das distale Ende des Nagels 1 ohne eingesetzten Spreizkörper parallel zum Schaft 7 ausgerichtet.

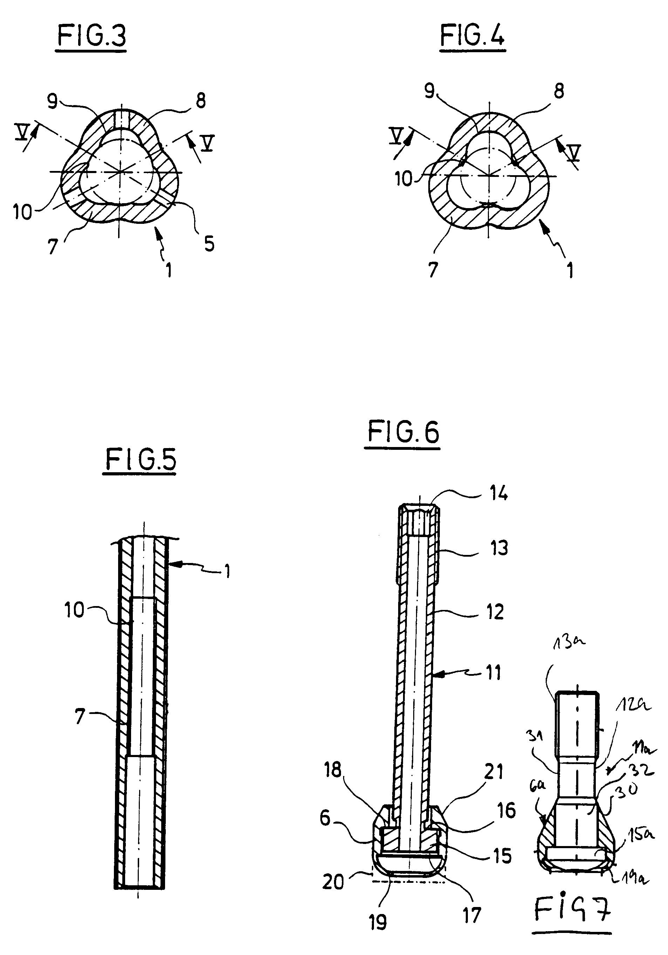

Den Fig. 3 und 4 ist entnehmbar, daß den Erhebungen 8 am Außenumfang Vertiefungen 9 am Innenumfang des Schaftes 7 entsprechen, zwischen denen am Innenumfang Erhebungen 10 angeordnet sind. Sie zeigen ferner, daß der von den Erhebungen 10 am Innenumfang begrenzte Innendurchmesser vom distalen Ende 4 des Nagels 1 zu dessen proximalem Ende hin abnimmt. Fig. 4 in Verbindung mit Fig. 5 zeigt außerdem, daß der Schaft 7 auf den Erhebungen 10 mit einem Innengewinde 11 versehen ist.

Ein vergleichbarer Nagel ist schon aus der EP-B-0 226 701 bekannt und kann proximal statt mit Verriegelungsscheiben auch mit einer Klammer versehen sein, die spinnenartig angeordnete Arme aufweist.

Zum Aufspreizen ist in den Nagel 1 eine Schraube 11 gemäß Fig. 6 eindrehbar, deren Schraubenschaft 12 einenends einen vergrößerten Durchmesser hat, der mit einem Gewindeabschnitt 13 versehen ist. Der Gewindeabschnitt 13 wirkt mit dem Innengewinde 10 des Nagels 1 zusammen. In dasselbe Ende des Schraubenschaftes 12 ist ein Innensechskant 14 eingearbeitet, in den durch das proximale Ende 2 des Nagels 1 ein Werkzeug einsetzbar ist.

Anderenends hat der Schraubenschaft 12 einen zylindrischen Schraubenkopf 15 mit einer dem Schraubenschaft 12 zugewandten Rückseite 16 und einer davon abgewandten Vorderseite 17.

Ein scheibenartiger Spreizkörper 6 liegt mit einer radial nach innen vorspringenden Stützfläche 18 an der Rückseite 16 des Kopfes 15 an und ist mit nur wenigen Zehntel Millimetern Spiel am Umfang des Kopfes 15 geführt. Der Spreizkörper 6 übergreift die Vorderseite 17 des Kopfes mit einer Einbördelung 19, die durch Umformen eines strichliert eingezeichneten Scheibenrandes 20 erzeugt ist. Der Kopf 15 ist so mit axialem Spiel zwischen Stützfläche 18 und Einbördelung 19 eingesetzt.

Schließlich hat der Spreizkörper 6 außen einen Konus 21, der sich zum Schraubenschaft 12 hin verjüngt.

Der Außendurchmesser des Spreizkörpers 6 stimmt etwa mit dem des unverformten Schaftes 7 des Nagels 1 überein und beträgt z.B. 9 oder 11 mm. Beim Eindrehen der Schraube 11 in das distale Ende 4 des Nagels 1 spreizt er das distale Ende auf, wobei der Konus 21 zusammen mit den Erhebungen 10 einen großen Spreizbereich garantiert. Bei einem äußeren Schaftdurchmesser von 9 mm kann eine Spreizung auf z.B. 18 mm erreicht werden.

Beim Aufspreizen gleitet die Rückseite 16 des Kopfes relativ reibungsarm auf der Stützfläche 18 des Spreizkörpers 6, die ihrerseits infolge der auf den Konus 21 wirkenden Reibkräfte nicht mitdreht. Beim Herausdrehen der Schraube 11 stellt die Einbördelung 19 die Mitnahme des Spreizkörpers 6 sicher. Im einfachsten Fall kann der Spreizkörper 6 eine Scheibe sein.

Die kürzere Spreizschraube 11a mit dem Schaft 12a und dem Kopf 15a weist ein Außengewinde 13a auf und kann ebenfalls einen Innnsechskant oder dergleichen haben zum Eingriff eines Drehwerkzeugs. Wie erkennbar ist eine drehbare Spreizkappe 6a vorgesehen, die einen konischen hinteren Abschnitt 30 aufweist, der sich von dem dünneren Schaftabschnitt 31 bis zur Unterseite des Kopfes 15a erstreckt. Der Kopf hat einen zylindrischen und einen balligen Abschnitt. Die Kappe 6a ist an den Abschnitt des Kopfes 6a angepaßt, während die Einwölbung 19a vom balligen Kopfabschnitt einen Abstand hat, wie auch in Fig. 6 gezeigt. Trotz der Verdrehbarkeit ist die Kappe fest und sicher von der Schraube geführt. Sowohl Schraube 11a als auch Kappe 6a sind sehr einfach herstellbar.

## Patentansprüche

1. Knochennagel zur Versorgung von Oberarmfrakturen, mit einem im proximalen Bereich in die Markhöhle einführbaren hohlen Schaft (7), der am distalen Ende (4) mehrere achsparallele Schlitze (5) und außerhalb des geschlitzten Bereichs ein Innengewinde (10) aufweist, und mit einer Schraube (11), deren Schraubenschaft (12) einen mit dem Innengewinde (10) zusammenwirkenden Gewindeabschnitt (13) und die im Kopfbereich einen Spreizkörper (6) aufweist, der durch Drehen der Schraube in den Schaft (7) hineinziehbar ist und das aufgeschlitzte Ende radial aufweitet, dadurch gekennzeichnet, daß der Spreizkörper (6) am Kopf (15) der Schraube (11) abgestützt und relativ zu diesem drehbar ist.

2. Knochennagel nach Anspruch 1, dadurch gekennzeichnet, daß der Spreizkörper (6) mit einer radial nach innen vorspringenden Stützfläche (18) an der dem Schraubenschaft (12) zugewandten Rückseite (16) des Kopfes (15) anliegt.

3. Knochennagel nach Anspruch 1 oder 2, dadurch gekennzeichnet, daß der Spreizkörper (6) die vom Schraubenschaft (12) abgewandte Vorderseite (17) des Kopfes (15) mit einem radial nach innen vorspringenden Rand, vorzugsweise mit einer Einbördelung (19), übergreift.

4. Knochennagel nach einem der Ansprüche 1 bis 3, dadurch gekennzeichnet, daß der Kopf (15) der Schraube (11) zwischen vorspringendem Rand (19) und Stützfläche (18) des Spreizkörpers (6) mit axialem Spiel eingesetzt ist.

5. Knochennagel nach einem der Ansprüche 1 bis 4, dadurch gekennzeichnet, daß der Spreizkörper (6) am Umfang der Schraube, vorzugsweise am Umfang ihres Kopfes (15), spielarm geführt ist.

6. Knochennagel nach einem der Ansprüche 1 bis 5, dadurch gekennzeichnet, daß der Kopf (15) der Schraube (11) zylindrisch ist.

7. Knochennagel nach einem der Ansprüche 1 bis 6, dadurch gekennzeichnet, daß der Spreizkörper (6) außen einen Konus (21) hat, der sich zum Schraubenschaft (12) hin verjüngt.

8. Knochennagel nach einem der Ansprüche 1 bis 7, dadurch gekennzeichnet, daß der hohle Schaft (7) innen in Umfangsrichtung abwechselnd Erhebungen (10) und Vertiefungen (9) hat, wobei der durch die Erhebungen definierte Innenradius im geschlitzten Bereich des Schaftes (7) vom distalen Ende (4) zum proximalen Ende (2) hin abnimmt.

9. Knochennagel nach Anspruch 7 oder 8, dadurch gekennzeichnet, daß auch der Konusbereich (30) des Spreizkörpers (6a) innen am Schraubenschaft (32) anliegt.

## Claims

1. A bone pin for treatment of a fractured upper arm, comprising a hollow shank (7) adapted to be inserted into the marrow cavity in the proximal area, which shank has at its distal end (4) a plurality of axially extending slots (5) and, outside of the slotted area, internal threads (10), and a bolt (11), the bolt shaft (12) of which has a threaded portion (13) cooperating with said internal threads (10) and which includes, at its head portion, an expansion element (6) which is adapted to be drawn into said shank (7) by rotation of the bolt so as to radially spread the slotted end, characterized in that the expansion element (6) is supported at the head (15) of the bolt (11) and is rotatable relatively thereto.

2. The bone pin of claim 1, characterized in that the expansion element (6) has a radially inwardly extending support surface (18) engage the backface (16) of the head (15) which faces the bolt shaft (12).

3. The bone pin of claim 1 or claim 2, characterized in that the expansion element (6) has a radially inwardly extending edge, preferably a crimped portion (19) extend about the front face (17) of the head (15) which faces away from the bolt shaft (12).

4. The bone pin of any of claims 1 to 3, characterized in that the head (15) of the bolt (11) is disposed with axial play between the inwardly extending edge (19) and the support surface (18) of the expansion element (6).

5. The bone pin of any of claims 1 to 4, characterized in that the expansion element (6) is guided with little play on the periphery of the bolt, preferably on the periphery of its head (15).

6. The bone pin of any of claims 1 to 5, characterized in that the head (15) of the bolt (11) is cylindrical.

7. The bone pin of any of claims 1 to 6, characterized in that the expansion element (6) has, on the outside, a conical portion (21) which tapers inwardly towards the bolt shaft (12).

8. The bone pin of any of claims 1 to 7, characterized in that the hollow shank (7) is internally provided with circumferentially alternating projections (10) and recesses (9), the projections defining an internal circle having a radius decreasing in the slotted area of the shank (7) from the distal end (4) to the proximal end (2).

9. The bone pin of claim 7 or claim 8, characterized in that the conical portion (30) of the expansion element (6a) internally engages the bolt shaft (32).

## Revendications

1. Clou destiné au traitement des fractures du bras, comportant:
une tige creuse (7) pouvant être introduite dans sa zone proximale dans la cavité médullaire, ladite tige présentant à son extrémité distale (4), plusieurs entailles (5) parallèles à l'axe et, en dehors de la zone entaillée, un filetage intérieur (10)
et une vis (11), dont la tige (12) présente un segment fileté (13) correspondant au filetage intérieur (10), et qui présente, au voisinage de la tête, un élément d'écartement (6), qu'on peut faire pénétrer dans la tige (7) en serrant le boulon, et qui écarte en direction radiale l'extrémité entaillée,
caractérisé en ce que l'élément d'écartement (6) s'appuie contre la tête (15) du boulon (11) et peut tourner par rapport à celle-ci.

2. Clou selon la revendication 1, caractérisé en ce que l'élément d'écartement (6) repose par une surface d'appui (18) faisant saillie radialement vers l'intérieur, sur la face arrière (16) de la tête (15),dirigée vers la tige du boulon (12).

3. Clou selon la revendication 1 ou 2, caractérisé en ce que l'élément d'écartement (6) recouvre la face avant (17) de la tête (15) opposée à la tige du boulon (12), par un rebord faisant saillie radialement vers l'intérieur, de préférence par un bord repoussé (19).

4. Clou selon l'une des revendications 1 à 3, caractérisé en ce que, la tête (15) du boulon (11) est mise en place avec un jeu axial entre le bord faisant saillie (19) et la surface d'appui (18) de l'élément d'écartement (6).

5. Clou selon l'une des revendications 1 à 4, caractérisé en ce que l'élément d'écartement (6) présente peu de jeu avec la périphérie de la vis, de préférence avec la périphérie de sa tête (15).

6. Clou selon l'une des revendications 1 à 5, caractérisé en ce que la tête (15) du boulon (11) est cylindrique.

7. Clou selon l'une des revendications 1 à 6, caractérisé en ce que l'élément d'écartement (6) comporte à l'extérieur un cône (21), qui se rétrécit vers la tige du boulon (12).

8. Clou selon l'une des revendications 1 à 7, caractérisé en ce que la tige creuse (7) comporte alternativement à l'intérieur en direction du périmètre, des bosses (10) et des creux (9), sachant que le rayon intérieur défini par les bosses dans la zone des entailles de la tige (7) diminue de l'extrémité distale (4) vers l'extrémité proximale (2).

9. Clou selon la revendication 7 ou 8, caractérisé en ce que la zone du cône (30) de l'élément d'écartement (6a) est aussi à l'intérieur en contact avec la tige du boulon (32).
